# EUROPEAN PATENT APPLICATION

(11) **EP 3 656 845 A1**
(43) Date of publication of application: **27.05.2020**
(21) Application number: 20150493.3
(22) Date of filing: 17.03.2016
(51) Int. Cl.: C12N 1/00, C12M 1/00, C12M 1/04, C12M 1/36, C12P 7/08, C12M 1/34

(54) **MICROORGANISM CULTURE METHOD AND CULTURE APPARATUS**

(30) Priority: 20.03.2015 JP 2015057463
(62) Divisional of application: 16768600.5
(71) Applicant: SEKISUI CHEMICAL CO., LTD., Osaka-shi Osaka 530-8565 (JP)
(72) Inventor: SATOU, Kanetomo, Tsukuba-shi, Ibaraki 300-4292 (JP); ISHII, Tetsuya, Tsukuba-shi, Ibaraki 300-4292 (JP)
(74) Representative: Ter Meer Steinmeister & Partner

(57) **Abstract**

The gas-utilizing microorganisms b are cultured in a liquid culture medium 9 that occupies a fermentative environment region 19 in a reaction tank 10 including a loop reactor 10 having a main tank portion 11 and a reflux portion 12B. A height of an upper communicating position of the main tank portion 11 communicating with the upper end portion of the reflux portion 12B is adjustable in stepless. The gas-utilizing microorganisms b can be fermented in the fermentative environment region 19. A substrate gas is supplied to the fermentative environment region 19 from a gas supply member 22. A volume of the fermentative environment region 19 is controlled by adjusting the height according to a supply flow rate of the substrate gas (g).

## Description

### Field of the Invention

The present invention relates to a method and an apparatus for culturing microorganisms and particularly relates to a culture method and a culture apparatus for culturing gas-utilizing microorganisms that fermentatively produce valuable materials such as ethanol from a substrate gas such as a synthetic gas.

### Background of the Invention

Some anaerobic microorganisms are known to produce valuable materials such as ethanol from a substrate gas by a fermentative action (refer to the patent documents listed below). The gas-utilizing microorganisms of this type may be cultured in a liquid culture medium. A typical culture tank may be of a stirred type, an airlift type, a bubble-column type, a loop type, a packed type, an open pond type, a photobiological type, or the like. A substrate gas may be a synthetic gas including, for example, CO, H₂, CO₂ or the like. The synthetic gas may be produced in a steel plant, a coal factory, a waste disposal facility, or the like. The synthetic gas may be supplied to the culture tank, thereby the gas-utilizing microorganisms may be made to ferment.

### Prior Art Documents

### Patent Documents

Patent Document 1: United States Patent No. 8,658,415
Patent Document 2: United States Patent Application Publication No. US2013/0065282
Patent Document 3: Japanese Patent Application Publication No. 2014-050406

### Summary of the Invention

### Problem to be Solved by the Invention

A gas supply flow rate from a synthetic gas source (substrate gas source) may not be constant. Particularly, in a case of a waste disposal facility, raw materials may include a wide variety of wastes, which may not be thoroughly segregated. Moreover, an amount of wastes may not be constant. Therefore, quantity of produced synthetic gas may not be stable. Moreover, sometimes operation of some of multiple treatment buildings may have to be suspended due to regular or irregular maintenance work or occurrence of trouble or the like. In such a case, the amount of gas supply flow rate may be greatly fluctuated, declining to a half in some cases.

When an amount of the synthetic gas supplied to a culture tank is not sufficient, generally all individuals of gas-utilizing microorganisms may be uniformly weakened and die. If generally all individuals of the gas-utilizing microorganisms died, they may need to be cultured again from an inoculum.

One measure to prevent such a situation may be to stockpile the synthetic gas in a reserve tank and provide the synthetic gas when necessary. However, a stockpile amount may be limited and may not be able to cope with a longstanding shortage of a supply of synthetic gas. Some suggests suppressing activity of the gas-utilizing microorganisms by cooling a culture medium (Patent Document 1). But it is only a temporary measure.

In view of the above, it is an object of the present invention to culture the gas-utilizing microorganisms in a stable manner even if the supply flow rate of the substrate gas such as the synthetic gas fluctuates.

### Means for Solving the Problems

To solve the problems mentioned above, a method of the present invention provides a culture method for culturing gas-utilizing microorganisms that produce valuable materials from a substrate gas by fermentation, the method including steps of: culturing the gas-utilizing microorganisms in a liquid culture medium that occupies a fermentative environment region in a reaction tank, the fermentation being allowed in the fermentative environment region; supplying the substrate gas to the fermentative environment region; and controlling a volume of the fermentative environment region according to a supply flow rate of the substrate gas.

The fermentative environment region mentioned above means a region that has an environment in which the gas-utilizing microorganisms can produce the valuable materials from the substrate gas by fermentation (fermentative environment). In other words, the fermentative environment region means a region in which an activity of the gas-utilizing microorganisms can be maintained. The fermentative environment region is a region that has a culture medium therein and a required amount of substrate gas can be supplied thereto.

According to this method, for example, when the supply flow rate of the substrate gas is decreased, the volume of the fermentative environment region is reduced. When the supply flow rate of the substrate gas is recovered, the volume of the fermentative environment region is increased to return to the original size. By this arrangement, the supply flow rate of the substrate gas in the fermentative environment region per unit volume can be maintained generally constant regardless of fluctuation of the substrate gas. Therefore, an amount of the substrate gas intake by each individual of the gas-utilizing microorganisms in the fermentative environment region can be stabilized. As a result, the gas-utilizing microorganisms can be cultured in a stable manner regardless of the fluctuation of the substrate gas. Thus, the gas-utilizing microorganisms can be prevented from dying due to a deterioration of a supply state of the substrate gas.

Preferably, the reaction tank includes a loop reactor having a main tank portion and a reflux portion, the method further including a step of: circulating the culture medium between the main tank portion and the reflux portion; and wherein a volume of a circulation region in the main tank portion and the reflux portion is controlled in the controlling step, the culture medium being circulated in the circulation region.

By this arrangement, the gas-utilizing microorganisms can be cultured in a stable manner.

Preferably, a communicating position from the main tank portion to the reflux portion is controlled in the controlling step.

By this arrangement, the volume of the circulation region can be controlled, and thereby, the volume of the fermentative environment region can be controlled.

Preferably, a volume changing member is advanceable into and retreatable from the reaction tank in the controlling step.

A volume of the reaction tank can be reduced by a volume corresponding to the advancement of the volume changing member into the reaction tank. When the volume changing member is retreated, the volume of the reaction tank is increased by a volume corresponding to the retreat. By this arrangement, the volume of the fermentative environment region can be surely increased or decreased.

An apparatus of the present invention provides a culture apparatus for culturing gas-utilizing microorganisms that produce valuable materials from a substrate gas by fermentation, including: a reaction tank having a fermentative environment region that allows the fermentation therein, the gas-utilizing microorganisms being cultured in a liquid culture medium that occupies the fermentative environment region; a gas supply member supplying the substrate gas to the fermentative environment region; and a volume changing mechanism, changing a volume of the fermentative environment region, wherein: the volume is controlled by the volume changing mechanism according to a supply flow rate of the substrate gas.

In this apparatus, for example, when the supply flow rate of the substrate gas is decreased, the volume of the fermentative environment region is reduced by the volume changing mechanism. When the supply flow rate of the substrate gas is recovered, the volume of the fermentative environment region is returned to the original size by the volume changing mechanism. By this arrangement, the gas-utilizing microorganisms can be cultured in a stable manner regardless of the fluctuation of the supply flow rate of the substrate gas.

Preferably, the reaction tank is a loop reactor having a main tank portion and a reflux portion, the culture medium being circulated between the main tank portion and the reflux portion; and wherein the volume changing mechanism changes a volume of a circulation region in the main tank portion and the reflux portion, the culture medium being circulated in the circulation region.

By this arrangement, the gas-utilizing microorganisms can be cultured in a stable manner.

Preferably, a communicating position from the main tank portion to the reflux portion is changeable.

By this arrangement, the volume of the circulation region can be controlled, and thereby, the volume of the fermentative environment region can be controlled.

Preferably, an intermediate portion of the main tank portion and an intermediate portion of the reflux portion are connected by one or a plurality of openable and closable connecting channels spaced from one another in a flow direction of the culture medium.

The communicating position from the main tank portion to the reflux portion can be changed by opening one connecting channel or by selectively opening one of the plurality of connecting channels. By this arrangement, the volume of the circulation region can be changed, and thereby, the volume of the fermentative environment region can be changed.

Preferably, the volume changing mechanism includes a volume changing member that changes the volume of the fermentative environment region by being advanced into and retreated from the reaction tank.

The volume of the reaction tank can be reduced by a volume corresponding to the advancement of the volume changing member into the reaction tank. When the volume changing member is retreated, the volume of the reaction tank is increased by a volume corresponding to the retreat. By this arrangement, the volume of the fermentative environment region can be surely increased or decreased.

Preferably, the volume changing mechanism includes: a bag expandable and shrinkable in the reaction tank; and a supplying/discharging mechanism adapted to supply and discharge a fluid pressure into and from the bag.

The bag can be expanded (advanced) by introducing a positive fluid pressure into the bag. The volume of the reaction tank can be reduced by a volume corresponding to the expansion of the bag, and thereby, the volume of the fermentative environment region can be reduced. The bag can be arranged to shrink (retreated) by eliminating or sucking a gas in the bag. The volume of the reaction tank can be increased by a volume corresponding to the shrinkage of the bag, and thereby, the volume of the fermentative environment region can be increased.

Preferably, the volume changing mechanism includes a rod member that is advanceable into and retractable from the reaction tank.

When the rod member is advanced into the reaction tank, the volume of the reaction tank can be reduced by a volume corresponding to the advancement of the rod member, and thereby, the volume of the fermentative environment region can be reduced. When the rod member is retreated from the reaction tank, the volume of the reaction tank can be increased by a volume corresponding to the retreat of the rod member, and thereby, the volume of the fermentative environment region can be increased.

Preferably, the volume changing mechanism includes a partition that divides an inside of the reaction tank into a chamber that is communicable with the gas supply member and a chamber that is shut-off from the gas supply member, wherein the shut-off chamber is releasable from the shut-off state by operation of the partition.

When the supply flow rate of the substrate gas declined, a portion (chamber) of the reaction tank is shut off from the gas supply member by a partition. Then, the shut-off chamber will not be a fermentative environment in which the gas-utilizing microorganisms can produce the valuable materials from the substrate gas by fermentation because the substrate gas is not supplied to the shut-off chamber. Therefore, the volume of the fermentative environment region can be reduced. Moreover, the substrate gas is supplied to the chamber that is communicable with the gas supply member, and thereby, the activity of the gas-utilizing microorganisms in the communicable chamber can be maintained. When the supply flow rate of the substrate gas is recovered (increased), the shut-off chamber is released from the shut-off state by operation of the partitions. Thereby, the substrate gas is supplied to the chamber released from the shut-off state. By returning the shut-off chamber to the fermentative environment, the volume of the fermentative environment region can be increased.

### Advantageous Effects of the Invention

According to the present invention, gas-utilizing microorganisms can be cultured in a stable manner even when a supply flow rate of a substrate gas fluctuates.

### Brief Description of the Drawings

FIG. 1 is an explanatory drawing showing a general configuration of a culture apparatus according to a first embodiment of the present invention in a normal operation mode.
FIG. 2 is an explanatory drawing showing the general configuration of the culture apparatus in a substrate gas insufficient supply mode.
FIG. 3 is an explanatory drawing showing a general configuration of a culture apparatus according to a second embodiment of the present invention.
FIG. 4 is an explanatory drawing showing a general configuration of a culture apparatus according to a third embodiment of the present invention. FIG. 4(a) shows the apparatus in a normal operation mode. FIG. 4(b) shows the apparatus in a substrate gas insufficient supply mode.
FIG. 5 is an explanatory drawing showing a general configuration of a culture apparatus according to a fourth embodiment of the present invention. FIG. 5(a) shows the apparatus in a normal operation mode. FIG. 5(b) shows the apparatus in a substrate gas insufficient supply mode.
FIG. 6 is an explanatory drawing showing a general configuration of a culture apparatus according to a fifth embodiment of the present invention. FIG. 6(a) shows the apparatus in a normal operation mode. FIG. 6(b) shows the apparatus in a substrate gas insufficient supply mode.

### Mode for Carrying out the Invention

Embodiments of the present invention will be described hereinafter with reference to the drawings.

### <First Embodiment>

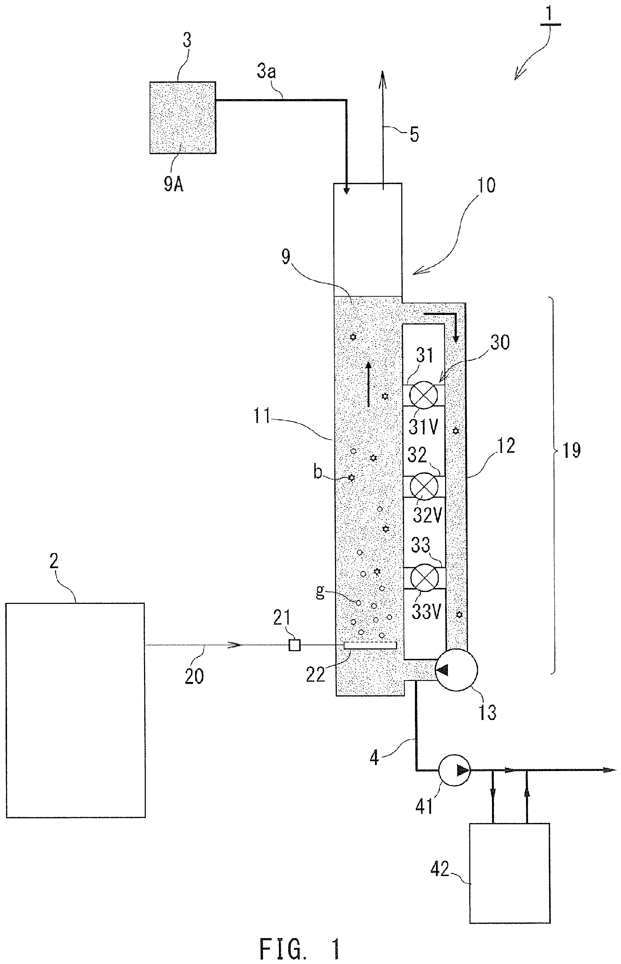

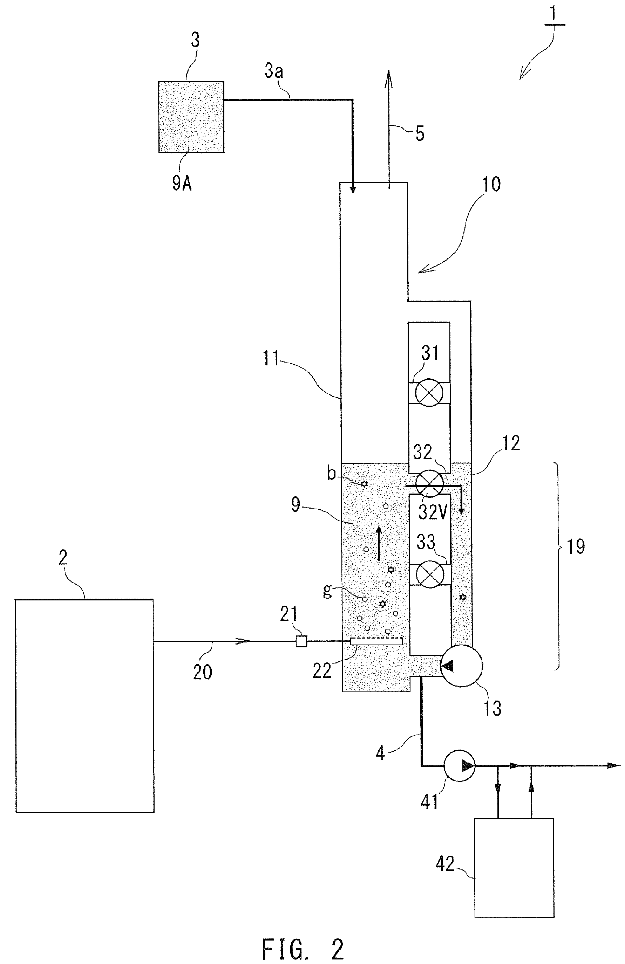

FIGS. 1 and 2 show a culture apparatus 1 according to a first embodiment of the present invention. Anaerobic gas-utilizing microorganisms b are cultured in the culture apparatus 1 as shown in FIG. 1. The gas-utilizing microorganisms b may include those disclosed in the patent documents listed above. The gas-utilizing microorganisms b fermentatively produce valuable materials (target substance) from a substrate gas g. The target substance of the apparatus 1 is ethanol (C₂H₅OH).

A synthetic gas (syngas) including CO, H₂ and CO₂ is used as the substrate gas g. The substrate gas g is produced in a substrate gas producing facility 2 (synthetic gas producing facility). The substrate gas producing facility 2 is a waste disposal facility in this embodiment. Wastes may include municipal wastes, tires, biomass, wooden chips and plastic wastes. The waste disposal facility is provided with a melting furnace. In the melting furnace, the wastes are burnt by a highly-concentrated oxygen gas and decomposed at a low-molecular level. Eventually, the substrate gas g (synthetic gas) including CO, H₂ and CO₂ is produced.

Required constituents of the substrate gas g can be selected as appropriate according to a kind of the gas-utilizing microorganisms b and the target substance. The substrate gas g may include only either one of the CO and H₂.

The culture apparatus 1 includes a loop reactor 10 as a reaction tank or a culture tank. The loop reactor 10 includes a main tank portion 11 and a reflux portion 12. The main tank portion 11 has a cylindrical configuration extending in a vertical (top-bottom) direction. A liquid culture medium 9 is received in the main tank portion 11. The liquid culture medium 9 is composed mostly of water (H₂O) with nutrient contents such as vitamins and phosphoric acid dissolved therein. The gas-utilizing microorganisms b are cultured in the liquid culture medium 9.

A fresh culture medium supply source 3 is connected to an upper end portion of the main tank portion 11 via a culture medium supply passage 3a. A fresh liquid culture medium 9A is stored in the fresh culture medium supply source 3. The gas-utilizing microorganisms b are not contained in the fresh liquid culture medium 9A.

A diffuser tube 22 (gas supply member) is disposed in a lower end portion of an inside of the main tank portion 11. A gas supply passage 20 extending from the substrate gas producing facility 2 is connected to the diffuser tube 22. A flow meter 21 is disposed in the gas supply passage 20. A pretreating portion such as a desulfurizing portion and a deoxidizing portion may also be disposed in the gas supply passage 20.

An exhaust passage 5 extends from the upper end portion of the main tank portion 11.

The reflux portion 12 has a tubular configuration extending vertically in parallel to the main tank portion 11. An upper end portion of the reflux portion 12 is connected to a side portion of the main tank portion 11 near an upper end of the main tank portion 11. A lower end portion of the reflux portion 12 is connected to a bottom portion of the main tank portion 11. A circulation pump 13 is disposed in the reflux portion 12.

In a normal operation mode (FIG. 1), a liquid level of the liquid culture medium 9 in the main tank portion 11 is at an upper side portion of the main tank portion 11. Specifically, the liquid level is higher than the upper end portion of the reflux portion 12. A portion of the main tank portion 11 from the bottom portion to the upper side portion thereof and the reflux portion 12 are filled with the liquid culture medium 9. And the liquid culture medium 9 is circulated between the main tank portion 11 and the reflux portion 12 by the circulation pump 13. A fermentative environment region 19 is provided by a region of the loop reactor 10 filled with the liquid culture medium 9 or a region of the loop reactor 10 in which the liquid culture medium 9 is circulated (circulation region). A required amount of the substrate gas g is supplied to the liquid culture medium 9 in the fermentative environment region 19. The gas-utilizing microorganisms b can fermentatively produce the valuable materials such as ethanol from the substrate gas g in the fermentative environment region 19.

The culture apparatus 1 further includes a volume changing mechanism 30 for changing a volume of the fermentative environment region 19. The volume changing mechanism 30 includes a plurality of connecting channels 31, 32, 33 and a send-out passage 4.

The plurality of connecting channels 31, 32, 33 are disposed between the main tank portion 11 and the reflux portion 12 spaced from each other in the vertical direction (flow direction of the culture medium 9). Intermediate portions of the main tank portion 11 and the reflux portion 12 in an extending direction are connected by the plurality of connecting channels 31, 32, 33.

Specifically, an upper-level connecting channel 31 connects the reflux portion 12 and a portion of the main tank portion 11 slightly below a portion connecting the main tank portion 11 to the upper end portion of the reflux portion 12. An on-off valve 31V is disposed in the connecting channel 31. The connecting channel 31 is opened and closed by the on-off valve 31V.

A middle-level connecting channel 32 connects the reflux portion 12 and a portion of the main tank portion 11 at a generally middle height. An on-off valve 32V is disposed in the connecting channel 32. The connecting channel 32 is opened and closed by the on-off valve 32V.

A lower-level connecting channel 33 connects the reflux portion 12 and a portion of the main tank portion 11 below the connecting channel 32. An on-off valve 33V is disposed in the connecting channel 33. The connecting channel 33 is opened and closed by the on-off valve 33V.

The number of the connecting channels of the volume changing mechanism 30 may not be three. There may be only one connecting channel or two connecting channels or four or more connecting channels in the volume changing mechanism 30.

The send-out passage 4 branches from a portion of the reflux portion 12 between the circulation pump 13 and the bottom portion of the main tank portion 11. A send-out pump 41 is disposed in the send-out passage 4. A buffer tank 42 is disposed along a path of the send-out passage 4 on a downstream side with respect to the send-out pump 41. Though not shown in the drawing, a downstream end of the send-out passage 4 extends to a subsequent treatment part such as a distiller. The send-out passage 4 has a function of sending the liquid culture medium 9 to the subsequent treatment part such as the distiller and a function as a composing element of the volume changing mechanism 30.

A method of culturing the gas-utilizing microorganisms b using the culture apparatus 1, and thereby a method of producing the valuable materials such as ethanol will be described hereinafter.

### <Normal Operation Mode>

Now, it is assumed that the culture apparatus 1 is in a normal operation mode as shown in FIG.1. The on-off valves 31V, 32V, 33V are all closed.

### <Substrate Gas Supplying Step>

The following description will be made on assumption that the waste disposal facility 2 is in a normal operation and a specified amount of the substrate gas g is produced. The substrate gas g is sent out to the diffuser tube 22 via the gas supply passage 20. The substrate gas g is supplied to the liquid culture medium 9 in the loop reactor 10, i.e. the fermentative environment region 19, from the diffuser tube 22. The substrate gas g is dissolved in the liquid culture medium 9 while being moved upward in the liquid culture medium 9 in the main tank portion 11.

### <Fermentation Step>

Then the gas-utilizing microorganisms b in the liquid culture medium 9 ingest CO and H₂ in the substrate gas g and ferment, thereby producing ethanol (valuable material). The produced ethanol becomes mixed in the liquid culture medium 9.

### <Circulating Step>

At the same time, the circulation pump 13 is activated. Thereby, the liquid culture medium 9 in the loop reactor 10 is circulated between the main tank portion 11 and the reflux portion 12. Specifically, the liquid culture medium 9 is moved upward in the main tank portion 11. Then, the liquid culture medium 9 enters the reflux portion 12 from the upper side portion of the main tank portion 11. Then the liquid culture medium 9 is moved downward in the reflux portion 12 and returned to the bottom portion of the main tank portion 11.

### <Send-Out Step>

A portion of the liquid culture medium 9 in the loop reactor 10 is sent out to the send-out passage 4.

### <Refining Step>

The portion of the liquid culture medium 9, after going through treatments such as solid-liquid separation, is distilled in a distillation tower that is not shown. Thereby, the ethanol is refined.

### <Replenishment Step>

The fresh liquid culture medium 9A in an amount corresponding to the amount of the liquid culture medium 9 sent out to the send-out passage 4 is replenished to the loop reactor 10 from the fresh culture medium supply source 3. By this arrangement, an amount of the liquid culture medium 9 in the loop reactor 10 can be maintained constant. Thereby, the volume of the fermentative environment region 19 can be maintained constant.

### <Exhaust Step>

Of the substrate gas g supplied to the loop reactor 10, an unused gas and a by-product gas produced by fermentation are exhausted from the exhaust passage 5 in the upper end portion of the main tank portion 11. The exhausted gas may be reused after going through processing such as impurity elimination.

### <Flow Rate Sensing Step>

A quantity of the substrate gas produced in the substrate gas producing facility 2 that is a waste disposal facility fluctuates greatly. A supply flow rate of the substrate gas g is sensed by the flowmeter 21.

### <Fermentative Environment Region 19 Volume Controlling Step>

The volume of the fermentative environment region 19 is controlled based on the sensed flow rate.

Specifically, when the supply flow rate of the substrate gas g is declined to the supply flow rate in the normal operation mode, the volume of the fermentative environment region 19 is made smaller. Thereby, the supply flow rate of the substrate gas g and the volume of the fermentative environment region 19 are made to correlate with each other.

The volume of the fermentative environment region 19 may be controlled automatically using a controller (control means). Alternatively, the volume of the fermentative environment region 19 may be manually controlled by an administrator.

### <Substrate Gas Insufficient Supply Mode>

It is assumed that the supply flow rate of the substrate gas g becomes a half thereof due to an occurrence of some kind of a trouble or maintenance work or the like at the substrate gas producing facility 2. In this case, as shown in FIG. 2, generally a half of the liquid culture medium 9 in the loop reactor 10 is discharged to the send-out passage 4. Thereby, the volume of the fermentative environment region 19 may become generally the half the volume thereof in the normal operation mode (FIG. 1). (The fresh liquid culture medium 9A in an amount corresponding to the amount of the discharged liquid culture medium 9 is not added from the fresh culture medium supply source 3.)

The discharged liquid culture medium 9 is stored in the buffer tank 42 to be taken out as appropriate and sent out to a distillation tower, etc.

### <Circulation Region Changing Step>

As shown in FIG. 2, a liquid level of the liquid culture medium 9 after discharging is at a position between the upper-level connecting channel 31 and the middle-level connecting channel 32, for example.

In response to this, the middle-level on-off valve 32V is opened. The upper-level on-off valve 31V and the lower-level on-off valve 33V are closed. Thereby, a communicating position from the main tank portion 11 to the reflux portion 12 is changed from a height of the upper end portion of the reflux portion 12 to a height of the middle-level connecting channel 32. The liquid culture medium 9 is circulated from the main tank portion 11 to the middle-level connecting channel 32 to the reflux portion 12 in this order. Therefore, a volume of the liquid culture medium 9 in the circulation region is reduced to generally a half thereof.

Depending on a degree of decline of the supply flow rate of the substrate gas g, the liquid level of the liquid culture medium 9 may be between the upper end portion of the reflux portion 12 and the upper-level connecting channel 31. Moreover, the connecting position from the main tank portion 11 to the reflux portion 12 may be made to be at a height of the upper-level connecting channel 31 by opening the upper-level on-off valve 31V. Thereby, the liquid culture medium 9 may be circulated from the main tank portion 11 to the upper-level connecting channel 31 and to the reflux portion 12 in this order.

Alternatively, depending on the degree of decline of the supply flow rate of the substrate gas g, the liquid level of the liquid culture medium 9 may be between the middle-level connecting channel 32 and the lower-level connecting channel 33. Moreover, the connecting position from the main tank portion 11 to the reflux portion 12 may be made to be at a height of the lower-level connecting channel 33 by opening the lower-level on-off valve 33V. Thereby, the liquid culture medium 9 may be circulated from the main tank portion 11 to the lower-level connecting channel 33 and to the reflux portion 12 in this order.

The supply flow rate of the substrate gas g per unit volume of the fermentative environment region 19 can be maintained generally constant regardless of the fluctuation of the substrate gas g by controlling volume of the fermentative environment region 19. A concentration of the gas-utilizing microorganisms b in the fermentative environment region 19 hardly varies before and after the volume control. Therefore, the amount of the substrate gas g that each of the gas-utilizing microorganisms b in the fermentative environment region 19 intakes can be stabilized. As a result, the gas-utilizing microorganisms b can be securely cultured regardless of the fluctuation of the substrate gas g. Thereby, death of the entire population of the gas-utilizing microorganisms b due to lack of the substrate gas can be prevented.

Activity of the gas-utilizing microorganisms b can be maintained at a sufficient level even if the supply flow rate of the substrate gas g continues to be half or lower than half the flow rate in the normal operation mode for a long period of time (longer than two days, for example).

### <Recovering Step>

When the supply flow rate of the substrate gas g from the substrate gas producing facility 2 is recovered to a level of the flow rate in the normal operation mode, the fresh liquid culture medium 9A is added to the loop reactor 10 from the fresh culture medium supply source 3. Thereby, as shown in FIG. 1, the liquid level of the liquid culture medium 9 is returned to the upper side portion of the main tank portion 11. Moreover, the on-off valve 32V and all the other on-off valves 31V, 33V are closed. Thereby, the liquid culture medium 9 is circulated in an entire length region of the main tank portion 11 and the reflux portion 12, thereby returning to the normal operation mode.

Addition of the fresh liquid culture medium 9A temporarily lowers the concentration of the gas-utilizing microorganisms b in the fermentative environment region 19. However, with sufficient supply of the substrate gas g to the increased liquid culture medium 9, the gas-utilizing microorganisms b will grow rapidly. Thereby, the concentration of the gas-utilizing microorganisms b can be recovered to a level before the addition of the fresh liquid culture medium 9A in a short period of time.

Other embodiments of the present invention will be described hereinafter. Same reference numerals are used in the drawings to designate parts that correspond to those in foregoing embodiments and description thereof will be omitted.

### <Second Embodiment>

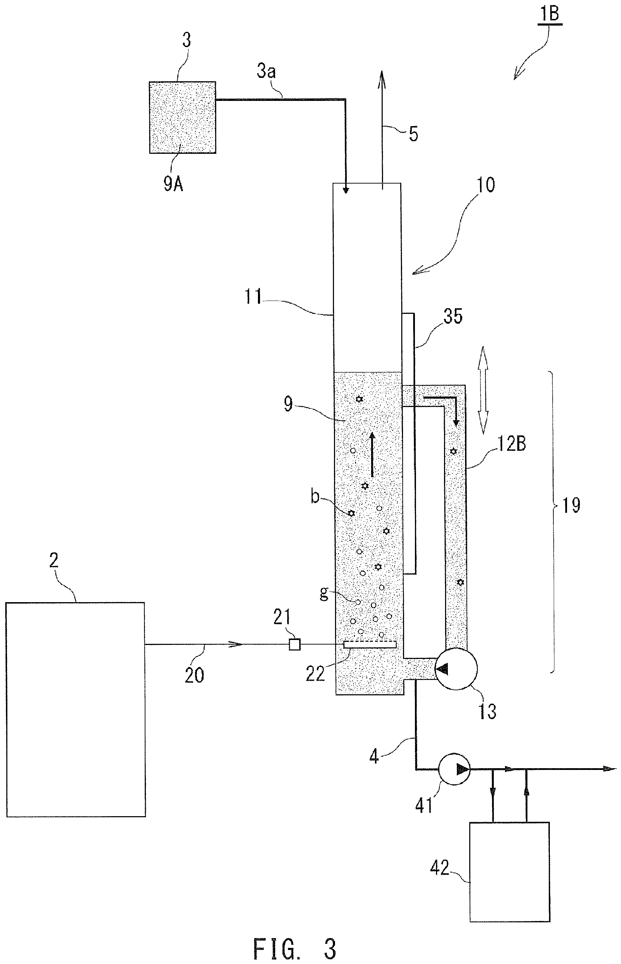

FIG. 3 shows a culture apparatus 1B according to a second embodiment of the present invention. In the culture apparatus 1B, instead of providing connecting channels 31, 32, 33, an upper end portion of a reflux portion 12B is adapted to be able to be lifted and lowered along a main tank portion 11. A sealing member 35 is provided between the upper end portion of the reflux portion 12B and an outer peripheral portion of the main tank portion 11. The sealing member 35 seals between the reflux portion 12B and the main tank portion 11 in a liquid-tight manner while allowing the upper end portion of the reflux portion 12B to be lifted and lowered. The reflux portion 12B is expandable and contractible accompanying lifting and lowering of the upper end portion. A flexible tube, a telescopic pipe, an accordion pipe or the like may be used as the reflux portion 12B that is expandable and contractible.

In the culture apparatus 1B, a height of an upper communicating position of the main tank portion 11 communicating with the reflux portion 12B can be adjusted in a non-stepwise fashion. Accordingly, a volume of a fermentative environment region 19 can be made to follow the change of the supply flow rate of the substrate gas g more accurately. As a result, an amount of the substrate gas that each of gas-utilizing microorganisms b in the fermentative environment region 19 intakes can be further surely stabilized. Thereby, the gas-utilizing microorganisms b can be securely cultured in a stable manner.

The height of the upper end portion of the reflux portion 12B may be controlled automatically using a controller based on a flow rate sensed by a flow meter 21 or may be controlled manually.

### <Third Embodiment>

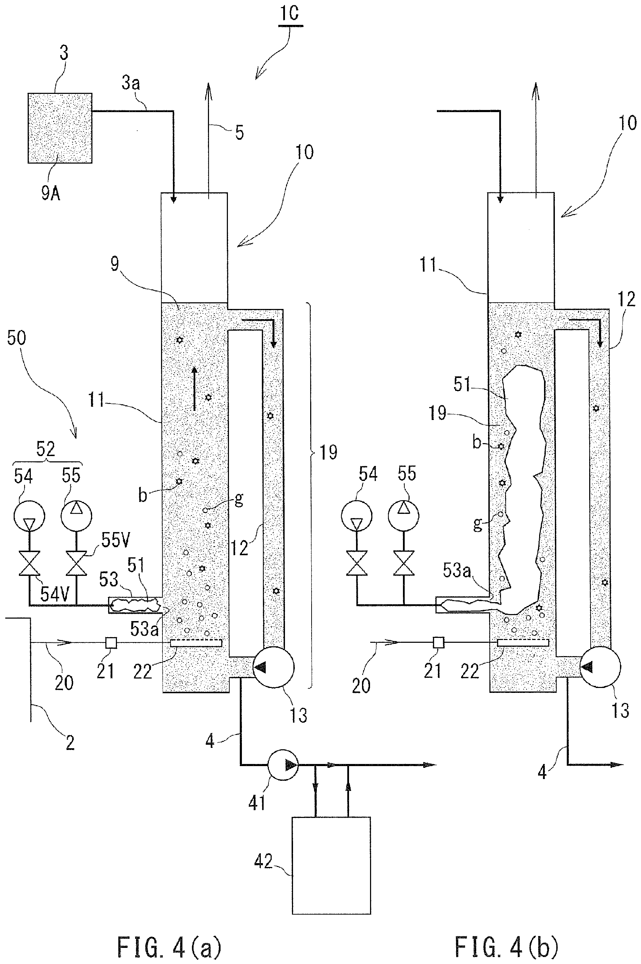

FIG. 4 shows a culture apparatus 1C according to a third embodiment of the present invention. A volume changing mechanism 50 of the culture apparatus 1C includes a bag 51 (volume changing member) and a supplying and discharging mechanism 52. The bag 51 is made of an air-tight resin film. Arrangement is made such that the bag 51 is expandable and shrinkable (advanceable and retreatable) in a loop reactor 10. A bag receiving portion 53 is provided in a side portion of a main tank portion 11 near a lower portion of the main tank portion 11. An inner portion of the bag receiving portion 53 is communicable with an inner portion of the main tank portion 11 through a communicating portion 53a.

The supplying and discharging mechanism 52 includes a compressor 54 and a vacuum pump 55. Arrangement is made such that by operation of on-off valves 54V, 55V, one of the compressor 54 and the vacuum pump 55 can be selectively communicable with the bag 51.

### <Normal Operation Mode>

As shown in FIG4 (a), in the normal operation mode, generally an entirety of the bag 51 is received in the bag receiving portion 53 in a shrunken state and the bag 51 faces an inside of the main tank portion 11 through the communicating portion 53a.

### <Substrate Gas Insufficient Supply Mode>

As shown in FIG. 4 (b), when a supply flow rate of a substrate gas g is declined, an air pressure (fluid pressure) is introduced to an inside of the bag 51 from the compressor 54. This causes the bag 51 to be advanced into the main tank portion 11 while being inflated. Preferably, an arrangement is made such that a degree of inflation of the bag 51 corresponds to a degree of decline of the supply flow rate of the substrate gas g. A liquid culture medium 9 in an amount corresponding to the inflation of the bag 51 is released from the loop reactor 10 to a send-out passage 4 and sent out to a buffer tank 42. Thereby, a volume of a fermentative environment region 19 is reduced. A liquid level of the liquid culture medium 9 in the main tank portion 11 is maintained generally constant. As a result, an amount of the substrate gas that each of gas-utilizing microorganisms b in the fermentative environment region 19 intakes can be stabilized regardless of fluctuation of the supply flow rate of the substrate gas g . Thereby, the gas-utilizing microorganisms b can be cultured in a stable manner.

As shown in FIG. 4 (a), when the supply flow rate of the substrate gas g is recovered, the vacuum pump 55 is activated to suction and exhaust air inside the bag 51. This causes the bag 51 to shrink and fit in the bag receiving portion 53. Thereby, the bag 51 is retreated from the main tank portion 11. Moreover, a fresh liquid culture medium 9A is added to the loop reactor 10 from a fresh culture medium supply source 3. Thereby, the volume of the fermentative environment region 19 can be increased to be recovered to a level of the normal operation mode. The gas-utilizing microorganisms b grow rapidly in the increased liquid culture medium 9. Thereby, the concentration of the gas-utilizing microorganisms b can be recovered to a level before the addition of the fresh liquid culture medium 9A in a short period of time.

Operation of the compressor 54 and the vacuum pump 55 to expand and shrink the bag 51 may be controlled automatically using a controller based on a flow rate sensed by a flow meter 21 or may be controlled manually.

### <Fourth Embodiment>

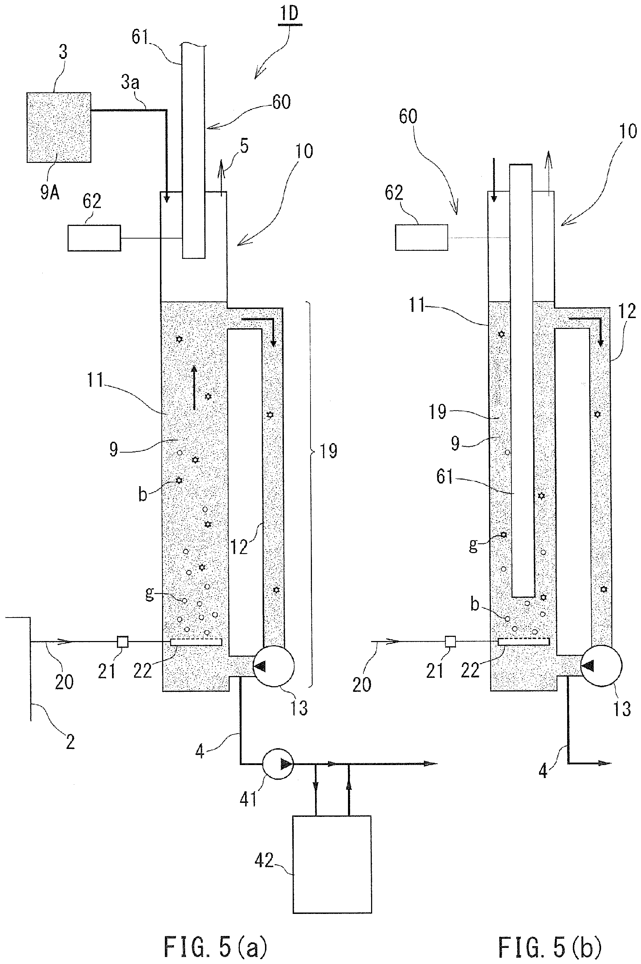

FIG. 5 shows a culture apparatus ID according to a fourth embodiment of the present invention. As shown in FIG. 5 (a), a volume changing mechanism 60 of the culture apparatus ID includes a rod member 61 (volume changing member) and a lifting and lowering driver 62. The rod member 61 extends straight in a vertical direction. The rod member 61 is arranged to be advanceable into and retreatable from the main tank portion 11 by being lifted and lowered along an axis of a main tank portion 11. The lifting and lowering driver 62 is connected to the rod member 61. Though not shown in detail in the drawings, the lifting and lowering driver 62 includes a motor and a slide guide. Lifted and lowered heights of the rod member 61 can be adjusted at any height by the lifting and lowering driver 62.

### <Normal Operation Mode>

As shown in FIG. 5 (a), the rod member 61 is at a lifted position in a normal operation mode. In this condition, a lower end portion of the rod member 61 is positioned higher than an upper end portion of a reflux portion 12 and positioned above a liquid level of a liquid culture medium 9 in a loop reactor 10.

### <Substrate Gas Insufficient Supply Mode>

As shown in FIG. 5 (b), when a supply flow rate of a substrate gas g is declined (substrate gas insufficient supply mode), the rod member 61 is lowered by the lifting and lowering driver 62. This causes the rod member 61 to be advanced into the main tank portion 11 and to enter the liquid culture medium 9. Preferably, an arrangement is made such that a degree of depth the rod member 61 enters the main tank portion 11 corresponds to a degree of decline of the supply flow rate of the substrate gas g. The liquid culture medium 9 in an amount corresponding to the degree of depth the rod member 61 enters is released to a send-out passage 4 and sent out to a buffer tank 42. Thereby, a volume of a fermentative environment region 19 is reduced. A liquid level of the liquid culture medium 9 in the main tank portion 11 is maintained generally constant. As a result, an amount of the substrate gas that each of gas-utilizing microorganisms b in the fermentative environment region 19 intakes can be stabilized regardless of fluctuation of the supply flow rate of the substrate gas g . Thereby, the gas-utilizing microorganisms b can be cultured in a stable manner.

As shown in FIG. 5 (a), when the supply flow rate of the substrate gas g is recovered, the rod member 61 is lifted by the lifting and lowering drive 62. Thereby, the rod member 61 is retreated above from the liquid culture medium 9 in the main tank portion 11. A fresh liquid culture medium 9A is added to the loop reactor 10 from a fresh culture medium supply source 3 in an amount corresponding to the retreat of the rod member 61. Thereby, the volume of the fermentative environment region 19 can be increased to be recovered to a level of the normal operation mode. The gas-utilizing microorganisms b grow rapidly in the increased liquid culture medium 9. Thereby, the concentration of the gas-utilizing microorganisms b can be recovered to a level before the addition of the fresh liquid culture medium 9A in a short period of time.

Operation of the lifting and lowering driver 62 to lift and lower the rod member 61 may be controlled automatically using a controller based on a flow rate sensed by a flow meter 21 or may be controlled manually.

After manually controlling a height of the rod member 61, the rod member 61 may be fixed to the main tank portion 11 with fixing means such as a screw.

### <Fifth Embodiment>

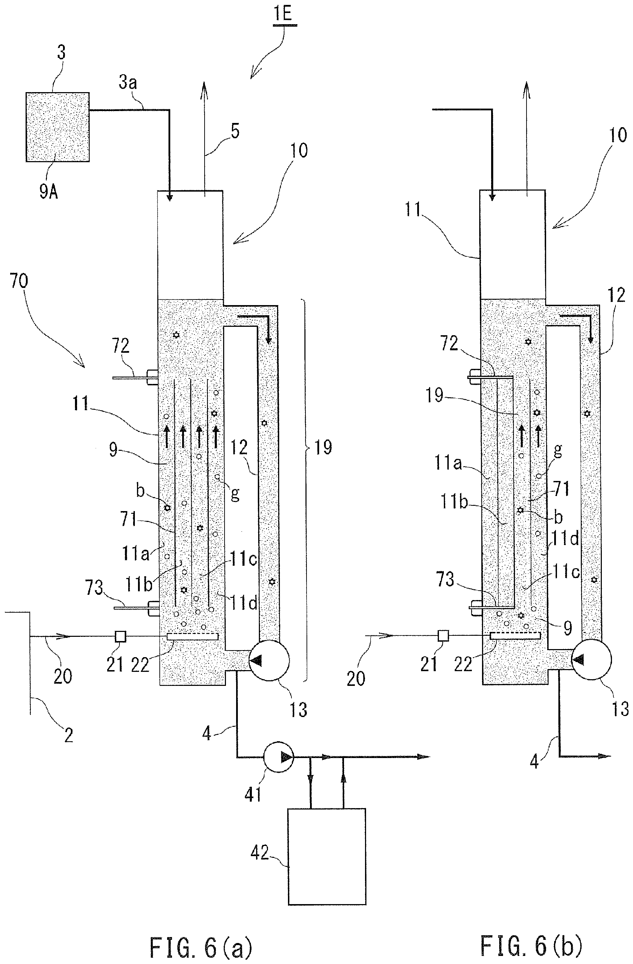

FIG. 6 shows a culture apparatus IE according to a fifth embodiment of the present invention. A volume changing mechanism 70 of the culture apparatus IE includes a plurality of fixed partitions 71 and two movable partitions 72, 73 (volume changing members). By these partitions 71, 72, 73, an inside of a loop reactor 10 can be divided into chambers 11c, 11d, etc. that are communicable with a diffuser tube 22 (gas supply member) and chambers 11a, 11b, etc. that are shut-off from the diffuser tube 22 (gas supply member). By operating these partitions 71, 72, 73, the chambers can be released from the shut-off state.

Specifically, as shown in FIG. 6 (a), the fixed partitions 71 are vertically disposed in an inside of a main tank portion 11. An upper end portion of each of the fixed partitions 71 is positioned slightly below an upper end portion of a reflux portion 12. A lower end portion of each of the fixed partitions 71 is positioned slightly above the diffuser tube 22. The main tank portion 11 is divided into a plurality of chambers 11a, 11b, 11c, 11d by the plurality of fixed partitions 71. Each of the chambers 11a, 11b, 11c, 11d extends vertically. A liquid level of a culture medium 9 in the main tank portion 11 is constantly positioned above the upper end portions of the fixed partitions 71, and thereby, above upper end portions of the chambers 11a, 11b, 11c, 11d.

The fixed partitions 71 may be parallel plates. The fixed partitions 71 may have a latticed configuration in a planar view or a radiated configuration in a planar view or a concentric circle configuration in a planar view or a combination of some of these configurations.

The two movable partitions 72, 73 are disposed in a side portion of the main tank portion 11 vertically spaced from each other. The movable partitions 72, 73 are horizontal plates. Each of the movable partitions 72, 73 can advance into and retreat from the main tank portion 11. As shown in FIG. 6(b), when the upper movable partition 72 is advanced into the main tank portion 11, upper end openings of one or more of the chambers 11a, 11b, 11c are closed depending on a degree of advancement of the upper movable partition 72. When the lower movable partition 73 is advanced into the main tank portion 11, lower end openings of one or more of the chambers 11a, 11b, 11c are closed depending on a degree of advancement of the lower movable partition 73. Preferably, the movable partitions 72, 73 are advanced or retreated (slid) in synchronization with each other.

Of the upper and lower movable partitions 72, 73, it is acceptable if at least the lower movable partition 73 is provided. The upper movable partition 72 may be omitted.

### <Normal Operation Mode>

As shown in FIG. 6 (a), the movable partitions 72, 73 are retreated outside of the main tank portion 11 in the normal operation mode. Thereby, upper and lower ends of all of the chambers 11a, 11b, 11c, 11d of the main tank portion 11 are opened. Lower end portions of the chambers 11a, 11b, 11c, 11d face the diffuser tube 22. By this arrangement, a substrate gas g is provided in the liquid culture medium 9 in the chambers 11a, 11b, 11c, 11d, thereby making the chambers 11a, 11b, 11c, 11d fermentative environment.

The liquid culture medium 9 is divided at a bottom portion of the main tank portion 11 to flow into the chambers 11a, 11b, 11c, 11d. The liquid culture medium 9 flows upward in the chambers 11a, 11b, 11c, 11d, exits the chambers 11a, 11b, 11c, 11d from upper end portions thereof and becomes confluent. After that, the liquid culture medium 9 is returned to the bottom portion of the main tank portion 11 via the reflux portion 12.

### <Substrate Gas Insufficient Supply Mode>

As shown in FIG. 6 (b), when a supply flow rate of the substrate gas g is declined, the movable partitions 72, 73 are advanced into the main tank portion 11 according to a degree of decline of the supply flow rate. Preferably, the upper and lower movable partitions 72, 73 are advanced into the main tank portion 11 to a same degree. By this arrangement, the upper and lower ends of some of the chambers 11a, 11b are closed. Therefore, these chambers 11a, 11b are shut off from the diffuser tube 22 (gas supply member), and the substrate gas g is not supplied to these chambers 11a, 11b. Moreover, the liquid culture medium 9 in the chambers 11a, 11b is confined in the chambers 11a, 11b. Therefore, the chambers 11a, 11b become non-fermentative environment, and the gas-utilizing microorganisms b in the chambers 11a, 11b can be dead. Of the chambers 11a, 11b, 11c, 11d, the substrate gas g from the diffuser tube 22 is supplied to the remaining chambers 11c, 11d. The liquid culture medium 9 that is not in the chambers 11a, 11b is circulated between the chambers 11c, 11d and the reflux portion 12. Therefore, the chambers 11c, 11d are maintained as the fermentative environment. In other words, a volume of a fermentative environment region 19 (circulation region) can be reduced. As a result, an amount of the substrate gas that each of the gas-utilizing microorganisms b in the fermentative environment region 19 intakes can be stabilized regardless of fluctuation of the supply flow rate of the substrate gas g. Thereby, the gas-utilizing microorganisms b can be cultured in a stable manner.

Depending on the degree of decline of the supply flow rate of the substrate gas g, a smaller number of the chambers 11a than in FIG. 6 (b) may be shut off. Alternatively, a greater number of the chambers 11a, 11b, 11c than in FIG. 6 (b) may be shut off.

In the culture apparatus IE of the fifth embodiment, an amount of the liquid culture medium 9 in the loop reactor 10 as a whole is maintained constant regardless of the supply flow rate of the substrate gas g. Therefore, a send-out passage 4 is not a composing element of the volume changing mechanism 70. The send-out passage 4 of the culture apparatus IE only plays a role of sending out the liquid culture medium 9 to a subsequent treatment part such as a distiller. In the fifth embodiment, a buffer tank 42 may be omitted.

As shown in FIG. 6 (a), when the supply flow rate of the substrate gas g is recovered, the movable partitions 72, 73 are retreated to the outside of the main tank portion 11. Thereby, the chambers 11a, 11b are released from the state of being shut off from the diffuser tube 22 (gas supply member). Thereby, the substrate gas g from the diffuser tube 22 enters the chambers 11a, 11b. Moreover, the liquid culture medium 9 becomes circulated between the chambers 11a, 11b and the reflux portion 12 as well. Accordingly, the chambers 11a, 11b return to be the fermentative environment, and thereby, the volume of the fermentative environment region 19 is increased. The gas-utilizing microorganisms b grow rapidly in the chambers 11a, 11b. Thereby, a concentration of the gas-utilizing microorganisms b can be rapidly restored to a predetermined level.

Operation to advance and retreat the movable partitions 72, 73 may be controlled automatically using a controller based on a flow rate sensed by a flow meter 21 or may be controlled manually.

The present invention is not limited to the embodiments described above. Various modifications can be made without departing from the scope and spirit of the invention.

For example, the substrate gas producing facility 2 is not limited to the waste disposal facility, but may be a steel plant or a coal factory.

The valuable materials are not limited to ethanol, but may be acetic acid, or the like.

In the culture apparatus 1, 1B, 1C, ID (FIG. 1 to 5), another discharge passage for discharging a part of the liquid culture medium 9 in the loop reactor 10 at the time of operation to reduce the volume of the fermentative environment region 19 may be provided in addition to the send-out passage 4 to the subsequent treatment part such as distiller.

Multiple embodiments may be combined. The loop reactor 10 may include the connecting channels 31, 32, 33 (FIG. 1) and the bag 51 (FIG. 4). The loop reactor 10 may include the connecting channels 31, 32, 33 (FIG. 1) and the rod member 61 (FIG. 5).

The loop reactor 10 may include the connecting channels 31, 32, 33 (FIG. 1) and the movable partitions 72, 73 (FIG. 6). In the first embodiment (FIG. 1, 2), movable partitions may be respectively disposed at heights slightly above the connecting channels 31, 32, 33. When the supply flow rate of the substrate gas g is declined, one of the connecting channels 31, 32, 33 may be opened according to the degree of decline and the main tank portion 11 may be divided by the movable partition immediately above the opened connecting channel. By this arrangement, a portion of the liquid culture medium 9 above the movable partition can remain there and at the same time can be excluded from the fermentative environment region 19. In this case, it is not required that the send-out passage 4 should be a composing element of the volume changing mechanism 30.

In the culture apparatus IE (FIG. 6) of the fifth embodiment, in place of the slidably movable partitions 72, 73, hingedly movable partitions may be rotatably disposed in an inner wall of the main tank portion 11 or in the upper and lower end portions of the fixed partition 71.

The loop reactor 10 may include the bag 51 (FIG. 4) and the rod member 61 (FIG. 5).

The reaction tank is not limited to the loop reactor 10, but may be a reaction tank of a type other than the loop type, such as stirred type, airlift type, bubble-column type, packed type, open pond type and photobiological type.

Multiple stages of the reaction tank may be provided.

In place of the flow rate of the entire supply gas suppled to the reaction tank, the volume of the fermentative environment region 19 may be controlled according to a flow rate of substrate constituents (substrate gas) in the supply gas such as CO and H₂.

### Industrial Applicability

The present invention may be applied to an ethanol generation system for synthesizing ethanol from carbon monoxide generated in an incineration treatment of industrial wastes, for example.

### Explanation of Reference Numerals

- b: gas-utilizing microorganisms
- g: substrate gas
- 1, 1B, 1C, 1D, 1E: culture apparatus
- 9: liquid culture medium (culture medium)
- 10: loop reactor (reaction tank)
- 11: main tank portion
- 12, 12B: reflux portion
- 19: fermentative environment region (circulation region)
- 22: diffuser tube (gas supply member)
- 30: volume changing mechanism
- 31, 32, 33: connecting channels
- 50: volume changing mechanism
- 51: bag (volume changing member)
- 52: supplying and discharging mechanism
- 60: volume changing mechanism
- 61: rod member (volume changing member)
- 70: volume changing mechanism
- 71: fixed partition (partition, volume changing member)
- 72: upper movable partition (partition, volume changing member)
- 73: lower movable partition (partition, volume changing member)
- 11a, 11b, 11c, 11d: chambers

## Claims

1. A culture method for culturing gas-utilizing microorganisms (b) that produce valuable materials from a substrate gas (g) by fermentation, **CHARACTERIZED IN THAT** the method comprises steps of:
culturing the gas-utilizing microorganisms (b) in a liquid culture medium (9) that occupies a fermentative environment region (19) in a reaction tank (10), the fermentation being allowed in the fermentative environment region (19), wherein the reaction tank (10) includes a loop reactor (10) having a main tank portion (11) and a reflux portion (12B), and wherein an upper end portion of the reflux portion (12B) is adapted to be able to be lifted and lowered, so that a height of an upper communicating position of the main tank portion (11) communicating with the upper end portion of the reflux portion (12B) is adjustable in stepless;
circulating the culture medium (9) between the main tank portion (11) and the reflux portion (12B);
supplying the substrate gas (g) to the fermentative environment region (19); and
controlling a volume of the fermentative environment region (19) by adjusting the height according to a supply flow rate of the substrate gas (g).

2. A culture apparatus (1B) for culturing gas-utilizing microorganisms (b) that produce valuable materials from a substrate gas (g) by fermentation, comprising:
a reaction tank (10) having a fermentative environment region (19) that allows the fermentation therein, the gas-utilizing microorganisms (b) being cultured in a liquid culture medium (9) that occupies the fermentative environment region (19); and
a gas supply member (22) supplying the substrate gas (g) to the fermentative environment region (19);
**CHARACTERIZED IN THAT** the reaction tank (10) is a loop reactor (10) having a main tank portion (11) and a reflux portion (12B) as a volume changing mechanism changing a volume of the fermentative environment region (19) according to a supply flow rate of the substrate gas (g), the culture medium (9) being circulated between the main tank portion (11) and the reflux portion (12B), and
wherein an upper end portion of the reflux portion (12B) is adapted to be able to be lifted and lowered, so that a height of an upper communicating position of the main tank portion (11) communicating with the upper end portion of the reflux portion (12B) is adjustable in stepless.

3. The culture apparatus (1B) according to claim 2, wherein a sealing member (35) is provided between the upper end portion of the reflux portion (12B) and an outer peripheral portion of the main tank portion (11), the sealing member (35) sealing between the reflux portion (12B) and the main tank portion (11) in a liquid-tight manner while allowing the upper end portion of the reflux portion (12B) to be lifted and lowered.
